# EUROPEAN PATENT APPLICATION

(11) **EP 4 763 113 A1**
(43) Date of publication of application: **24.06.2026**
(21) Application number: 25226806.5
(22) Date of filing: 23.12.2025
(51) Int. Cl.: A61B 17/72, A61B 17/80

(54) **FIXATION PLATE AND SYSTEM OF STABILIZING FRACTURED BONE**

(30) Priority: 23.12.2024 US 202463738086 P
(71) Applicant: Stryker European Operations Limited, Carrigtwohill T45H X08 (IE)
(72) Inventor: ZANDER, Nils, 24340 Eckernförde (DE); WIELAND, Manfred, 24146 Kiel (DE); KLUEVER, Hendrik, 24232 Schoenkirchen (DE); KELLY, Michael, Bristol, BS9 2RR (GB); KUBIAK, Erik, Las Vegas, 89135 (US); MACEROLI, Michael, Portage, 49002 (US); SEGINA, Daniel, Portage, 49002 (US); WESTRICK, Edward, Gibsonia, 15044 (US); YOON, Richard, Tenafly, 07670 (US)
(74) Representative: Maiwald GmbH

(57) **Abstract**

A fixation plate for securing bone fragments includes a first portion extending along a first longitudinal axis, a second portion extending from an end of the first portion along a second longitudinal axis such that the second longitudinal axis is at an angle of eighty to one hundred forty degrees relative to the first longitudinal axis, and first and second holes having respective first and second central hole axes that are substantially transverse to each other, wherein the first hole is disposed at a junction between the first and second portions, and the second hole is disposed at an end of the second portion opposite from the junction.

## Description

### CROSS-REFERENCE TO RELATED APPLICATIONS

The present application claims priority to U.S. Application No. 63/738,086 filed December 23, 2024, the disclosure of which is incorporated herein by reference.

### BACKGROUND OF THE INVENTION

The distal, condylar end of the femur has a trapezoid shaped anatomy and articulates with both the tibial plateau and the patella. The Hoffa fractures of the distal femur are injuries described as a coronal fracture of the femur involving one or both of the condyles. These fractures are quite rare, often going unobserved during the routine assessment of a distal femur fracture. As most Hoffa fractures occur bilaterally or laterally, the medial side, especially as an isolated incidence, appears to be of lesser importance. Generally, due to the high energy mechanism, Hoffa fractures are mostly seen as a part of a more complex distal femur condition. Hoffa fractures are considered articular fractures which require careful articular anatomical reduction and stable fixation. Nonoperative treatment can lead to fracture displacement and limited knee joint function.

Accordingly, to improve the stabilization potential of small medial Hoffa fragments, improvements are desired.

### BRIEF SUMMARY OF THE INVENTION

To improve the stabilization potential of small medial Hoffa fragments, a plate design such as for the medial side of the femur is provided which has an increased posterior footprint providing screw locking options distal-posteriorly allowing a surgeon to place a locking screw into the posterior aspect of the medial condyle. Such posteriorly placed locking screw can be inserted in a medial-lateral orientation to ensure fixation within the fragmented bone piece. Additionally, the plate offers a locking configuration which allows the placement of 2 (angular stable) locking screws into the medial posterior condyle in two planes of fixation, which in some embodiments may be perpendicular to each other, thus maximizing stabilization potential especially in poor bone quality. The variable angle locking mechanism allows a wide range of relative angulation between both locking screws in both the transversal and the sagittal plane. Additionally, the medial plates might offer the options for mechanical interlinking with a retrograde femur nail utilizing a hole in the plate. In some embodiments, the hole may be an oblong locking hole to compensate for anatomical variations during implant positioning.

The plate described herein and the related embodiments accommodate a long bone such as the femur by providing a stable construct that can be fixed to the shaft of the bone along with and anterior just a portion of the bone, while providing a contoured section of the plate that facilitates secures fixation of posterior bone fragments at the distal end of the femur. The contouring of the plate, which can take on the form of an L-shape or a 7-shape, provides unique access to fragmented portions of a femur suffering from Hoffa fractures. The positioning of different portions of the plate that provide access to both the anterior and posterior portions of the distal femur, as well as providing angled insertion of locking screws to enhance fixation, creates a unique solution to healing a bone subject to a Hoffa fracture. In particular, the contouring of the plate toward the posterior side of the bone permits insertion of a fixing screw in the medial-lateral direction, which is supplemented by additional fixation through the plate in the anterior-posterior direction. These two directions of fixation in coordination with one another creates a robust and secure solution to Hoffa fracture healing.

A first aspect of the present disclosure is a method of stabilizing fragments of a distal femur, including placing a fixation plate against the distal femur, securing a first portion of the fixation plate to a shaft of the distal femur, securing a second portion of the fixation plate to a condyle of the distal femur, and inserting a first fastener through a first hole of the fixation plate disposed at a junction between the first and second portions of the fixation plate, wherein securing the second portion of the fixation plate includes inserting a second fastener through a second hole in the second portion of the fixation plate. The first and second fasteners are substantially transverse to each other, and/or distal ends of the first and second fasteners are disposed in the same bony fragment of the condyle.

In accordance with the first aspect, the same bony fragment of the condyle may be a posterior fragment of the condyle. The second hole may be disposed at an end of the second portion opposite from the junction. The first fastener may be inserted into the distal femur at an angle of ten degrees to forty-five degrees relative to a bone facing surface of the fixation plate at the first hole, and the second fastener may be inserted into the distal femur at an angle substantially transverse to the bone facing surface of the fixation plate at the second hole. The steps of securing and inserting may stabilize one or more fragment of the condyle with respect to the remainder of the distal femur.

The method may further include inserting a third fastener through an elongated slot of the second portion. The method may further include inserting a third fastener through a hole of the second portion. The method may further include interlinking the third fastener with an intramedullary nail located within an intramedullary canal of the distal femur. The method may further include rotating the fixation plate about the third fastener disposed within the elongated slot or the hole so as to align the first portion of the fixation plate to the shaft of the distal femur. The step of securing the first portion of the fixation plate may include inserting a fastener through a hole of the first portion of the fixation plate and into the shaft of the distal femur.

A second aspect of the present disclosure is a method of stabilizing fragments of a distal femur, including placing a fixation plate against the distal femur, inserting a first fastener through a first hole of the fixation plate and into two distinct portions of a fractured bone, and inserting a second fastener through a second hole of the fixation plate and into only one of the two distinct portions of the fractured bone. In accordance with the second aspect, only one of the two distinct portions may be a bony fragment of the distal femur. The first and second fasteners may secure the same bony fragment in two planes.

A third aspect of the present disclosure is a method of stabilizing fragments of a distal femur, including placing a fixation plate against the distal femur, inserting a first fastener through a first hole of the fixation plate and into a fractured bone across a fracture line of the bone, and inserting a second fastener through a second hole of the fixation plate and only into a fractured portion of the fractured bone. In accordance with the third aspect, the fractured portion of the fractured bone may be a bony fragment of the distal femur. The first and second fasteners may secure the same bony fragment in two planes.

A fourth aspect of the present disclosure is a fixation plate for securing bone fragments, the fixation plate including a first portion extending along a first longitudinal axis, a second portion extending from an end of the first portion along a second longitudinal axis such that the second longitudinal axis is at an angle of eighty to one hundred forty degrees relative to the first longitudinal axis, and first and second holes having respective first and second central hole axes that are substantially transverse to each other, wherein the first hole is disposed at a junction between the first and second portions, and the second hole is disposed at an end of the second portion opposite from the junction.

In accordance with the fourth aspect, an entire bone facing surface of the fixation plate at the second portion may be concave. The entire bone facing surface of the fixation plate at the second portion may be substantially spherical. A bone facing surface of the fixation plate may be substantially configured to mate against a surface of a distal femur. The first central hole axis may be at an angle of ten degrees to forty-five degrees relative to a bone facing surface of the fixation plate at the first hole, and the second central hole axis may be substantially transverse to the bone facing surface of the fixation plate at the second hole. The second portion may further include an elongated slot. The second portion may further include a hole. The fixation plate may further include first and second fasteners fixed through the respective first and second holes in a substantially transverse orientation to each other. The second portion may further include an elongated slot, and the fixation plate may further include a third fastener fixed through the elongated slot. The second portion may further include a hole, and the fixation plate may further include a third fastener fixed through the hole.

A bone fixation system may include the aforementioned fixation plate and an intramedullary nail. The intramedullary nail may be a retrograde femur nail. The bone fixation system may further include first and second fasteners fixed through the respective first and second holes in a substantially transverse orientation to each other. The second portion may further include an elongated slot, and the system may further include a third fastener fixed through the elongated slot and through a hole in the intramedullary nail. The second portion may further include a hole, and the system may further include a third fastener fixed through the hole and through a hole in the intramedullary nail.

### BRIEF DESCRIPTION OF THE DRAWINGS

FIG. 1 is a top perspective view of a bone fixation system installed on a bone in accordance with an embodiment of the present disclosure.
FIG. 2 is a side perspective view of the system shown in FIG. 1.
FIG. 3 is a top perspective view of the system shown in FIG. 1.
FIG. 4 is a side perspective view of the system shown in FIG. 1.
FIG. 5 is a top perspective view of a bone fixation system installed on a bone in accordance with another embodiment of the present disclosure.
FIG. 6 is a side perspective view of the system shown in FIG. 5.

### DETAILED DESCRIPTION

Particular embodiments of the present disclosure will be described herein with reference to the accompanying drawings. As shown in the drawings and as described throughout the following description, and as is traditional when referring to relative positioning on an object, the term "proximal" means closer to a patient's heart and the term "distal" means further away from the patient's heart. The term "anterior" means toward the front of the patient's body and the term "posterior" means toward the back of the patient's body. The term "inferior" means toward the feet and the term "superior" means towards the head. The term "medial" means toward the midline of the body and the term "lateral" means away from the midline of the body. Also, as used herein, the terms "substantially," "generally," and "about" are intended to mean that slight deviations from absolute are included within the scope of the term so modified.

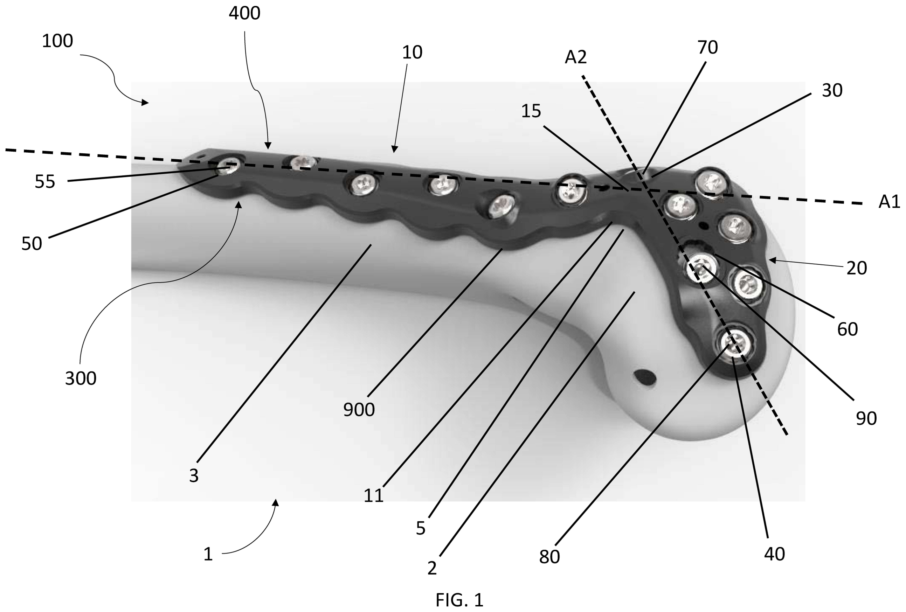

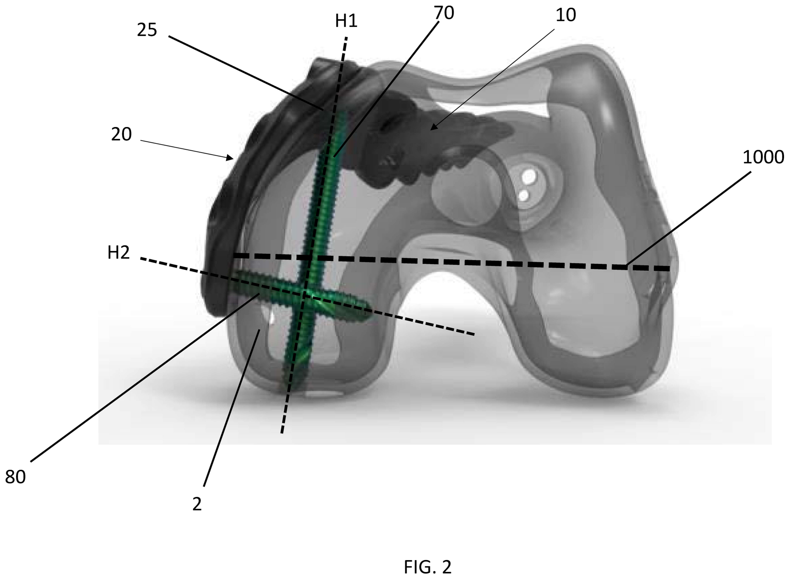

FIGS. 1 and 2 illustrate respective top and side perspective views of a fixation plate 100 designed for securing bone fragments in accordance with an embodiment of the present disclosure. Although the embodiments depicted herein describe and illustrate a femur, it is envisioned that the fixation plate may be implemented with other bones, including long bones such as the humerus, tibia, etc.

As illustrated in FIG. 1, the fixation plate 100 includes a first portion 10, which extends along a first longitudinal axis A1, and a second portion 20, which extends from an end of the first portion 10 along a second longitudinal axis A2. In other words, first portion 10 is generally linear to define first longitudinal axis A1, and second portion 20 is also generally linear to define second longitudinal axis A2. These linear configurations are understood when viewing the relevant portion from a direction perpendicular to the surface, and do not require the plate itself to be flat. In this way, plate 100 generally takes on the shape of an "L" or a "7". The second longitudinal axis A2 of the second portion 20 extends at an angle 5 of ninety to one hundred forty degrees relative to the first longitudinal axis A1 of the first portion 10. In some embodiments, the angle 5 may be from one hundred to one hundred twenty degrees, or from eighty to one hundred forty degrees. The angle 5 may be such that plate 100 is substantially configured to stabilize bone fragments of a distal femur. Further, the lengths and thicknesses of the first portion 10 and the second portion 20 along the first and second longitudinal axes A1 and A2, respectively, are not particularly limited, so long as the lengths and thicknesses are substantially configured to mate against a distal femur and to provide the requisite stability to the bone with which they are used.

As further shown in FIG. 1, an intersection of the first portion 10 and the second portion 20 defines a junction 15. More specifically, the junction 15 may be an area of the fixation plate 100 where the first longitudinal axis A1 and the second longitudinal axis A2 intersect. The junction 15 is the location at which the plate 100 transitions from the first portion 10 and the second portion 20, or in other words, where the first portion 10 and the second portion 20 are considered to overlap each other. This is directly adjacent to a concave portion 11 on the outer surface at an elbow where the first portion 10 and the second portion 20 meet. In accordance with the present disclosure, the fixation plate 100 further includes a first hole 30 and a second hole 40. The first hole 30 is disposed at the junction 15 such that it coincides with the intersection of axes A1 and A2, or is within a distance of such intersection, the distance being one length of the diameter of first hole 30. First hole 30 is directly adjacent to concave portion 11 at the elbow of the plate 100 so that first hole 30 is in a portion of plate 100 where first and second portions 10, 20 intersect. The second hole 40 is disposed at an end of the second portion 20 opposite from the junction 15. The first and second holes 30, 40 may be configured to accept fasteners known in the art. The second hole 40 being located at an end of the section portion 20 provides an optimal location for bone screw fixation since it is located further from the junction 15, and so permits the first and second holes 30, 40 to facilitate access to areas of the distal femur where screws can be inserted substantially transverse or perpendicular to each other. This gives the ability for screw placement in a posterior location of the distal end of the bone given the extension of the second portion 20 on the distal end of the plate 100.

As most clearly illustrated in FIG. 2, the first hole 30 has a first central hole axis H1, and the second hole 40 has a second central hole axis H2. Such central axes are defined and oriented with respect to the generally cylindrical inner side walls of the respective holes. The first and second central hole axes H1 and H2 are substantially transverse or perpendicular to each other when projected onto a horizontal plane perpendicular to the longitudinal axis of the femur on which plate 100 is attached. In other words, second central hole axis H2 is substantially perpendicular to the portion of plate 100 through which it extends, while first central hole axis H1 is angled in a direction toward second hole 40. Stated differently, in a plane containing first central hole axis H1 and parallel to second central hole axis H2, a projection of second central hole axis H2 onto the plane is substantially transverse or perpendicular to first central hole axis H1. The same relationship in a plane is true for the axes of the screws through the respective holes 30, 40. Since a screw or fastener will be inserted through each of the first and second holes 30, 40, first and second central hole axes H1 and H2 are not necessarily oriented to intersect. While first and second central hole axes H1 and H2 may intersect, they may also be askew so that they pass each other in a transverse or perpendicular relationship or orientation. In this way, screws can be inserted through first and second holes 30, 40 to be positioned in a posterior distal aspect of a femoral condyle. Plate 100 can be contoured for use with either the medial or lateral condyle of either the left or right femur.

As shown in FIG. 1, the fixation plate 100 further includes a bone facing surface 300 and a top facing surface 400. The bone facing and top facing surfaces 300, 400 are disposed on opposite sides of the fixation plate 100 and are generally parallel to the first and second longitudinal axes A1, A2, understanding that contouring for the bone surface is present. In some embodiments, the bone facing surface 300 may be configured to mate against a distal femur. More particularly, the bone facing surface 300 may be configured to mate against a shaft and a condyle of a distal femur.

The bone facing surface 300 of the first portion 10 and the second portion 20 may have respective first and second radii of curvature. In such embodiments, the second radius of curvature may be greater than the first radius of curvature, or vice versa, or the radii may be equal. Furthermore, in some embodiments, the bone facing surface 300 at the first portion 10 may be substantially cylindrical, and the bone facing surface 300 at the second portion 20 may be substantially spherical. In such embodiments, the fixation plate 100 may be configured to substantially mate against a bone by having a footprint matching the posterior portion of the bone. More particularly, the fixation plate may be configured to mate against a shaft and a condyle of a distal femur. In some embodiments, an entire bone facing surface 300 of the fixation plate 100 at the second portion 40 is concave. In such embodiments, the fixation plate 100 may substantially mate against a medial or lateral surface of a distal femur. In that regard, the plate 100 can be configured for use with the medial or lateral side of a bone, such as the femur. In other embodiments of the present disclosure, it is also envisioned that the bone facing surface 300 of the first portion 10 has both a convexity and a concavity. Specifically, the bone facing surface 300 of the first portion 10 substantially near the junction 15 may be concave, and a remainder of the bone facing surface 300 of the first portion 10 may be convex. In such embodiments, the fixation plate may be configured to substantially mate against a distal femur. In yet another embodiment, the entire bone facing surface 300 at the first portion 10 is concave.

As shown in FIG. 2, the first central hole axis H1 is at an angle 25 of ten degrees to forty-five degrees relative to the bone facing surface 300 at the first hole 30, i.e., to a plane tangent to the bone facing surface 300 at the first hole 30. Further, the second central hole axis H2 is substantially transverse or normal to the bone facing surface 300 of at the second hole 40, i.e., to a plane tangent to the bone facing surface 300 at the second hole 40.

The fixation plate 100 may further include a plurality of fixation holes 50 disposed along the first portion 10 and the second portion 20. The plurality of fixation holes 50 may be configured to accept fasteners known in the art. Further, in accordance with the present disclosure, an outer peripheral surface 900 of the fixation plate 10 that connects the bone facing and top facing surfaces 300, 400 may define grooves or be scalloped about the perimeter of plate 100. However, the present disclosure is not limited thereto, and the outer peripheral surface 900 may define any shape known in the art so long as the fixation plate 10 can mate against a distal femur.

As most clearly illustrated in FIG. 1, the fixation plate 100 may further include an oblong- or obround-shaped elongated slot 60 located within the second portion 20 of the fixation plate 100. The slot 60 may be configured to accept a fastener known in the art. Further, in such embodiments, the slot may have a length, in a direction substantially parallel to the second longitudinal axis A2, greater than a diameter of any one or more of the plurality of fixation holes 50. In other embodiments, slot 60 may not be present and may be omitted or replaced with a standard circular hole, as described below.

According to another aspect of the present disclosure, the fixation plate 100 may further include a first fastener 70 fixed through the first hole 30 and a second fastener 80 fixed through the second hole 40. As shown in the perspective of FIG. 2 generally along the axis of the femur, the first and second fasteners 70, 80, inserted substantially along the respective first and second central hole axes H1, H2, are substantially transverse to each other in their implanted configuration. In such embodiments, stabilization potential can be maximized, especially in poor quality bones. Further, a variable angle locking mechanism may allow for a wide range of relative angulation between the first and second fasteners 70, 80 in both the transversal and the sagittal planes. Accordingly, in such embodiments, the first and second fasteners 70, 80 may be configured to stabilize bone fragments of a condyle of a distal femur. In embodiments in which slot 60 is present, a third fastener 90 may be fixed through the elongated slot 60. In such embodiments, the third fastener 90 may be configured to further stabilize a condyle of a distal femur. The third fastener 90 may be configured to interlink with an intramedullary nail. Further, the type of fasteners used is not particularly limited, and may be, but is not limited to, bone screws, beams, pegs, nails, which may be threaded or non-threaded.

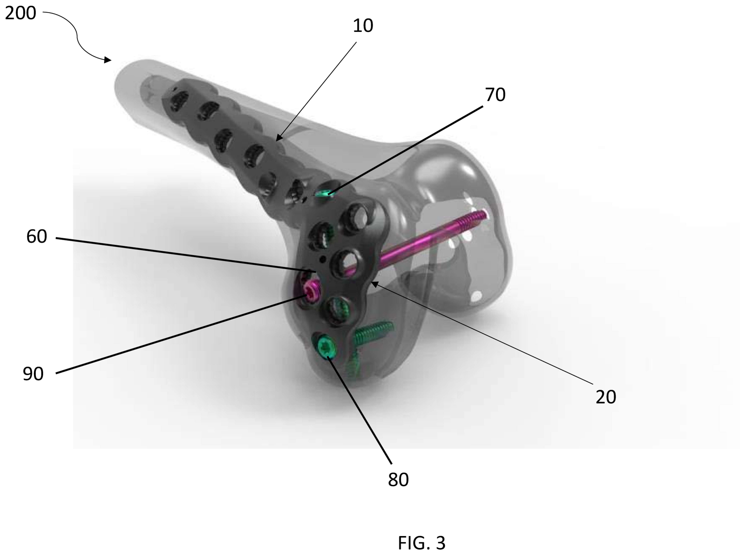

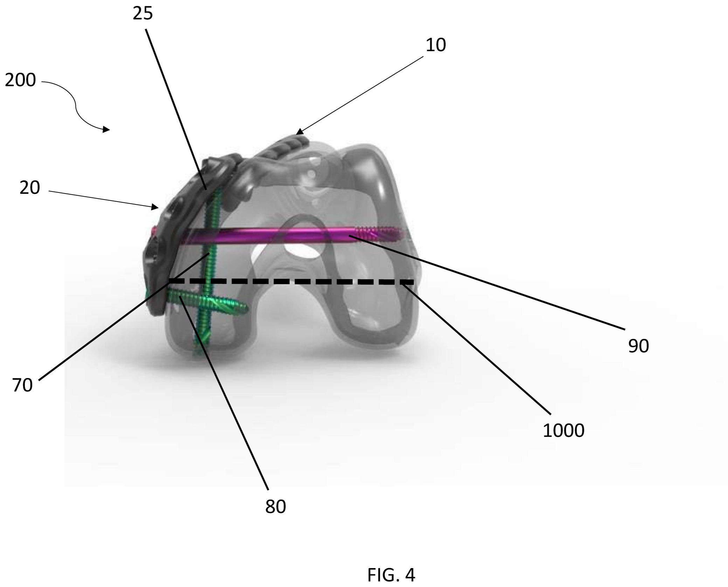

In another aspect of the present disclosure, as shown in FIGS. 3 and 4, a bone fixation system 200 includes the fixation plate 100 for use with an intramedullary nail. A third fastener 90 may be fixed through the elongated slot 60. The third fastener 90 may interlink with the intramedullary nail located within an intramedullary canal of the distal femur via a hole in the intramedullary nail.

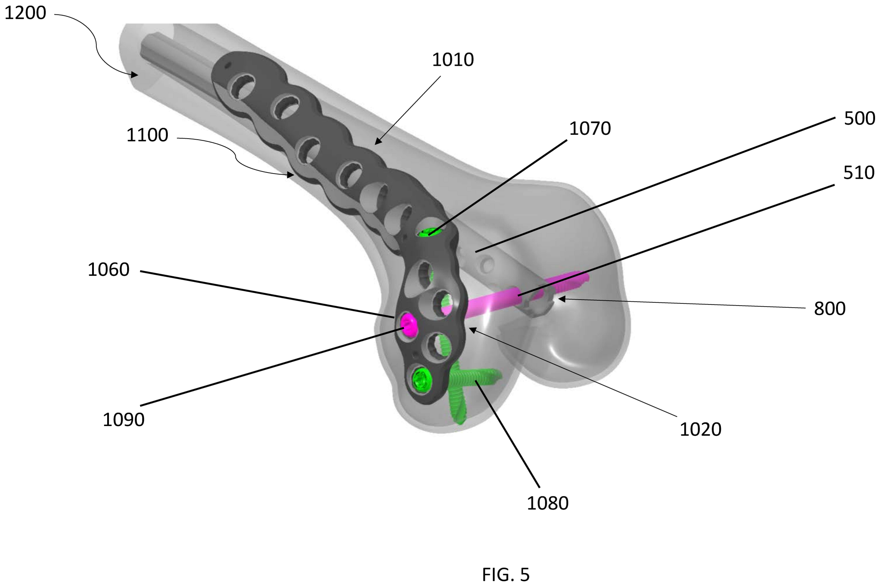

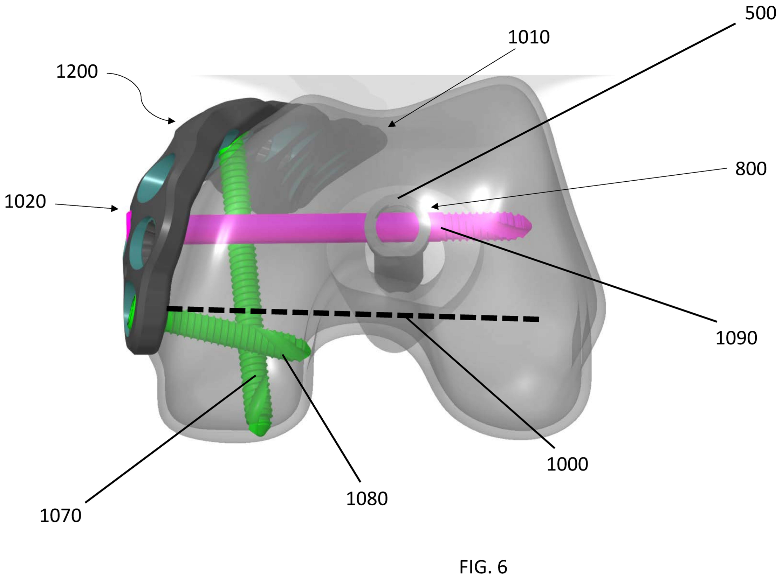

Another embodiment of a fixation system 1200 is shown in FIGS. 5 and 6. System 1200 includes a fixation plate 1100 for use with an intramedullary nail 500. Plate 1100 is similar to plate 100 with like elements numbered similarly. The intramedullary nail 500 may be any intramedullary nail known in the art. Preferably, the intramedullary nail 500 may be a retrograde femur nail or an antegrade femur nail when used with the femur. Like some embodiments of the fixation plate 1100, the bone fixation system may further include a first fastener 1070 fixed through the first hole disposed at the junction. Similarly, the bone fixation system 1200 may further include a second fastener 1080 fixed through the second hole disposed at an end of the second portion 1020 opposite from the junction. As in some embodiments of the fixation plate 1100, the first and second fasteners are substantially transverse to each other in their implanted configuration. While in other embodiments of the fixation plate 1100, the second portion 1020 of the fixation plate 1100 may further include an elongated slot, plate 1100 includes a circular hole 1060 instead. A third fastener 1090 may be fixed through the circular hole 1060. The third fastener 1090 may interlink with the intramedullary nail 500 via a hole 510 in the intramedullary nail.

A method of stabilizing fragments of a distal femur will now be described in the context of the system 1200 explained above. The method includes placing a bone facing surface of a fixation plate 1100 against a surface of a bone such as the distal femur 1, and on the medial or lateral side thereof depending on the need and the configuration of the plate 1100. This can be done using any means of guiding the fixation plate 1100, such as through the use of one or more k-wires. A first portion 1010 of the fixation plate 1100 is secured to a shaft 3 of the distal femur, and a second portion 1020 of the fixation plate 1100 is secured to a medial or lateral condyle 2 of the distal femur 1. These securing steps can be performed in either order. As described in detail in connection with FIGS. 5 and 6, the steps of securing include inserting a first fastener 1070 and a second fastener 1080 into a first hole and a second hole, respectively, along substantially transverse trajectories. As indicated, this transverse orientation is achieved by the first and second fasteners 1070, 1080 being askew so that they pass each other in a transverse or perpendicular relationship or orientation. In such a configuration, the ends of both the first and second fasteners 1070, 1080 are anchored in the same bony fragment of the bone in order to secure such fragment to the remaining bone using forces along two perpendicular axes. Specifically, as shown in FIG. 6, the steps of securing may stabilize bone fragments of the condyle of the distal femur with a fracture line 1000 substantially like those shown in FIGS. 2, 4, and 6. As seen in FIG. 6, the second fastener 1080 is disposed "beneath" the fracture line 1000 when viewed in this distal-to-proximal direction of the femur, *i.e.* in a horizontal plane, so that second fastener 1080 can capture the fragment of the bone. This ability of the present system to secure the fragment of the bone with second fastener 1080 as well as with a distal end of first fastener 1070 from a substantially perpendicular angle creates a more secure and robust fixation of the bone fragments to promote and enhance healing.

The steps of inserting the fasteners include inserting the first fastener 1070 into the bone at an angle of ten degrees to forty-five degrees relative to the bone facing surface of the fixation plate 1100 at the first hole. Also, the second fastener 1080 is inserted into the bone at an angle substantially transverse to the bone facing surface of the fixation plate 1100 at the second hole. Because of the curvature of the second portion 1020 with respect to the first portion 1010, this permits the substantially transverse or perpendicular orientation of the implanted first and second fasteners 1070, 1080. Thus, the plate 1100 provides for an increased anterior footprint on the femur, and a more vertical (anterior to posterior) screw trajectory to target medial or lateral Hoffa fragments. The fasteners can be inserted in any order, and the denotation of one fastener as a "first" does not require that it must be inserted "first".

The method may further includes inserting the third fastener 1090 through the circular hole 1060 (or the elongated slot 60 if present) of the second portion 1020. This further stabilizes bone fragments of the condyle 2 of the distal femur 1. In other embodiments, the third fastener 1090 or any fourth or additional fastener can be inserted through an additional circular hole in plate 100.

In some embodiments, as described in detail in connection with FIGS. 5 and 6, the method may further include interlinking the third fastener 1090 with an intramedullary nail 500 located within an intramedullary canal 800 of the distal femur 1 via a hole 510 in the nail 500. This can be done through using one or more aiming guides connected to a distal end of the nail 500 and/or to the plate 1100 that assist in alignment of the third fastener 1090 with the hole 510 in the nail 500. In this way, stronger fixation between the implanted nail 500 and the implanted plate 1100 can be achieved to expedite healing of the patient.

In another embodiment, the method may include first inserting third fastener 1090 within hole 1060 (or slot 60) or another hole in the plate 100, and then rotating and/or aligning the fixation plate 1100 as necessary about the third fastener 1090 so as to align the first portion 1010 of the fixation plate 1100 to the appropriate position on the shaft 3 of the distal femur. The step of rotating may compensate for anatomical variations during implant positioning. This may be aided when the use of a slot 60 provides additional movement of the plate during this process to properly fit the bone surface.

It is to be understood that this method may differ based on surgeon preference, location of the fracture lines, or the needs of the patient. Specifically, the order of insertion may differ for first fastener 1070, second fastener 1080, third fastener 1090, and fixation screw 55 in their respective holes.

In another embodiment of the method, a fixation plate is placed against the distal femur or other long bone. A first fastener is inserted through a first hole of the fixation plate and into two distinct portions of a fractured bone. These distinct portions can be the intact portion of the bone along with a bony fragment that has separated from the intact portion of the bone. In this way, the first fastener is inserted across a fracture line of the bone to secure the bony fragment to the intact portion of the bone. A second fastener is inserted through a second hole of the fixation plate and into only one of the two distinct portions of the fractured bone, i.e. the bony fragment. Thus, the second fastener connects the bony fragment to the plate without passing through the intact portion of the bone. In this way, the first and second fasteners secure the same bony fragment in two planes. This creates stabilization of the bony fragment and secures it in place against the intact portion of the bone so that it is prevented from rotation and held in a fixed position with forces from multiple angles to promote accurate alignment and thorough healing.

In other embodiments, the second or an additional fastener can also pass through both the intact portion of the bone as well as the bone fragment.

The foregoing methodology may involve the use of standard tools, such as screw drivers and drills to prepare the bone and place the screws. Guides, such as drill and screw placement guides, can also be utilized. This is particularly true in connection with embodiment plates having variable angle screw holes. The use of guides can dictate the placement of the screws according to the orientations described above.

It is to be understood that the disclosure set forth herein includes any possible combinations of the particular features set forth above, whether specifically disclosed herein or not. For example, where a particular feature is disclosed in the context of a particular aspect, arrangement, configuration, or arrangement, that feature can also be used, to the extent possible, in combination with and/or in the context of other particular aspects, arrangements, configurations, and arrangements of the technology, and in the technology generally.

Furthermore, although the technology herein has been described with reference to particular features, it is to be understood that these features are merely illustrative of the principles and applications of the present technology. It is therefore to be understood that numerous modifications, including changes in the sizes of the various features described herein, may be made to the illustrative arrangements and that other arrangements may be devised without departing from the spirit and scope of the present technology. In this regard, the present technology encompasses numerous additional features in addition to those specific features set forth in the claims below. Moreover, the foregoing disclosure should be taken by way of illustration rather than by way of limitation as the present technology is defined by the claims set forth below.

## Claims

1. A fixation plate for securing bone fragments, the fixation plate comprising:
a first portion extending along a first longitudinal axis;
a second portion extending from an end of the first portion along a second longitudinal axis such that the second longitudinal axis is at an angle of eighty to one hundred forty degrees relative to the first longitudinal axis; and
first and second holes having respective first and second central hole axes that are substantially transverse to each other,
wherein the first hole is disposed at a junction between the first and second portions, and the second hole is disposed at an end of the second portion opposite from the junction.

2. The fixation plate of claim 1, wherein an entire bone facing surface of the fixation plate at the second portion is concave.

3. The fixation plate of claim 2, wherein the entire bone facing surface of the fixation plate at the second portion is substantially spherical.

4. The fixation plate of any one of claims 1-3, wherein a bone facing surface of the fixation plate is substantially configured to mate against a surface of a distal femur.

5. The fixation plate of any one of claims 1-4, wherein the first central hole axis is at an angle of ten degrees to forty-five degrees relative to a bone facing surface of the fixation plate at the first hole, and the second central hole axis is substantially transverse to the bone facing surface of the fixation plate at the second hole.

6. The fixation plate of any one of claims 1-5, wherein the second portion further comprises an elongated slot.

7. The fixation plate of any one of claims 1-6, wherein the second portion further comprises a hole.

8. The fixation plate of any one of claims 1-5, further comprising first and second fasteners fixed through the respective first and second holes in a substantially transverse orientation to each other.

9. The fixation plate of claim 8, wherein the second portion further comprises an elongated slot, and further comprising a third fastener fixed through the elongated slot.

10. The fixation plate of claim 8, wherein the second portion further comprises a hole, and further comprising a third fastener fixed through the hole.

11. A bone fixation system, comprising:
the fixation plate of any one of claims 1-10; and
an intramedullary nail.

12. The bone fixation system of claim 11, wherein the intramedullary nail is a retrograde femur nail.

13. The bone fixation system of claim 11 or claim 12, further comprising first and second fasteners fixed through the respective first and second holes in a substantially transverse orientation to each other.

14. The bone fixation system of any one of claims 11-13, wherein the second portion further comprises an elongated slot, and further comprising a third fastener fixed through the elongated slot and through a hole in the intramedullary nail.

15. The bone fixation system of any one of claims 11-13, wherein the second portion further comprises a hole, and further comprising a third fastener fixed through the hole and through a hole in the intramedullary nail.
